# EUROPEAN PATENT APPLICATION

(11) **EP 4 248 944 A1**
(43) Date of publication of application: **27.09.2023**
(21) Application number: 22382276.8
(22) Date of filing: 25.03.2022
(51) Int. Cl.: A61K 9/00, A61K 31/366, A61K 31/728, A61K 47/36, A61P 31/02, A61P 31/04

(54) **ANTISEPTIC COMPOSITION CONTAINING 9-HYDROXYCALABAXANTHONE**

(71) Applicant: Universitat de les Illes Balears, 07122 Palma de Mallorca (ES); Fundació Institut d'Investigació Sanitària Illes Balears (IdISBa), 07120 Palma de Mallorca (ES)
(72) Inventor: MONJO CABRER, Marta, 07122 Palma de Mallorca (ES); RAMIS MOREY, Joana Maria, 07122 Palma de Mallorca (ES); VARGAS ALFREDO, Nelson, 07120 Palma de Mallorca (ES); MUNAR BESTARD, Marta, 07120 Palma de Mallorca (ES)
(74) Representative: Hoffmann Eitle

(57) **Abstract**

The present invention relates to a cosmetic or pharmaceutical composition containing 9-hydroxycalabaxanthone in the form of gel, aqueous solution, soap, or other presentations, which can be used efficiently in the antiseptic treatment of the buccal cavity, as well as in the disinfection of surfaces such as body surfaces, for example, in the treatment of medical conditions associated with infection (such as acute or chronic wounds, burns, and surgical wounds), or disinfection of the skin before a surgical procedure.

## Description

### FIELD OF THE ART

The present invention relates to a cosmetic or pharmaceutical composition containing 9-hydroxycalabaxanthone in the form of gel, aqueous solution, soap, or other presentations, which can be used efficiently in the antiseptic treatment of the buccal cavity, as well as in the disinfection of surfaces such as body surfaces.

### BACKGROUND OF THE INVENTION

Iodine, chlorhexidine, alcohol, acetate, hydrogen peroxide, boric acid, silver nitrate, silver sulfadiazine, and sodium hypochlorite are among the most widely used commercialized antiseptic agents in clinical practice. However, despite being excellent antiseptics, many of them present significant biocompatibility issues when they come into contact with living tissues.

In the dental field, there are many products on the market today for the treatment of the buccal cavity, and more specifically for the prevention and treatment of periodontitis, gingivitis, and for antiseptic protection after periodontal and/or peri-implant surgeries, after tooth extractions, or after the placement of dental implants. These formulations mainly consist of a physical mixture between high molecular weight natural substances for forming gels and an active substance with antimicrobial properties. Additionally, regenerative and/or anti-inflammatory properties are sought in many products. The most widely used products are hyaluronic acid (HA) as a gelling substance which also promotes tissue regeneration and chlorhexidine (CHX) as an antimicrobial substance, among others. These systems work suitably under certain conditions; however, they have some drawbacks. On one hand, the main drawbacks result from the very nature of the manufacture thereof. Since they are gels formed solely by means of the physical interaction of polymer chains, i.e., formed by means of physical gelling, they are very unstable in aqueous media. This results in frequent dosing of the treatment and the inability to use same as a prolonged release system. On the other hand, another characteristic of some of these systems is that they depend on CHX or other substances as the antimicrobial active ingredient. Although CHX has excellent antimicrobial properties, harmful effects on human gingival tissues have recently been reported. Furthermore, it has other side effects which also limit its use including, among them, impairment of the sense of taste, staining of teeth, and even peeling of oral mucosa.

For this reason, it is important to have a better alternative which has regenerative and antimicrobial properties without cytotoxic effects on human cells, and which is being released in a controlled and prolonged manner at the site of action.

### BRIEF DESCRIPTION OF THE FIGURES

Figure 1. Structure of 9-hydroxycalabaxanthone.
Figure 2. Effect of different concentrations of HCX on the activity of LDH released into the medium (A), metabolic activity (B), and morphology (C) of gingival fibroblasts. *p<0.05 treatment vs. C-; # p<0.05 treatment vs. CHX; &p<0.05 treatment vs. 100 µM HCX.
Figure 3. Effect of different concentrations of HCX on the growth (figure 3a) and live/dead ratio (figure 3b) of P. *gingivalis.* *p<0.05 treatment vs. C-; # p<0.05 treatment vs. CHX; & p<0.05 treatment vs. 100 µM HCX.
Figure 4. Characterization by means of ¹H NMR in deuterated water (D₂O) at 300 MHz of the chemical modification of HA with adipic acid dihydrazide (ADH). (A) HA before modification and (B) obtaining HA-ADH.
Figure 5. Characterization by means of ¹H NMR in deuterated water (D₂O) at 300 MHz of the chemical modification of HA with aldehyde groups (CHO). (A) HA before modification and (B) obtaining HA-CHO.
Figure 6. (A) Characterization of the swelling of modified HA gels and (B) cumulative release of HCX from a HA gel with 3 mM HCX.
Figure 7. Effect of modified HA gels with different concentrations of HCX on the growth (figure 7a) and live/dead ratio (figure 7b) of P. *gingivalis.* *p<0.05 treatment vs. HA; # p<0.05 treatment vs. HA 0.2% CHX; $ p<0.05 treatment vs HA 1.5 mM HCX.
Figure 8. MTT viability assay in 3D gingival tissue after the application of HA gels with different concentrations of HCX. *p<0.05 treatment vs. HA; # p<0.05 treatment vs. HA 0.2% CHX.

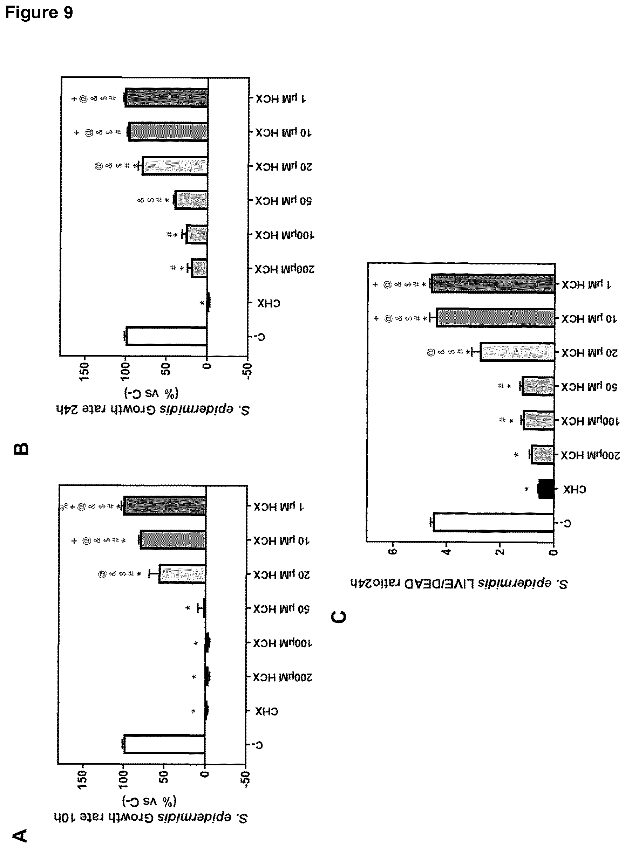

Figure 9. Antimicrobial activity of different concentration of HCX (A)S. epidermidis growth rate cultured at 10h with different concentration of HCX. (B)S. epidermidis growth rate cultured at 24h with different concentration of HCX. (C) S. epidermidis Live/Dead Ratio cultured for 24h with different concentration of HCX. Results are expressed as % vs negative control that was set to 100%. Data represents the mean ± SEM (n=6). Negative control (C-) was bacterial suspension without any treatment and positive control (C+) was bacterial suspension with CHX at 10µM. Results were statistically compared by ANOVA and Bonferroni as post hoc: *P<0.05 Treatment vs. C-. # P<0.05 Treatment vs. CHX. $ P<0.05 Treatment vs. 200µM HCX. & P<0.05 Treatment vs. 100µM HCX. @ P<0.05 Treatment vs. 50µM HCX. + P<0.05 Treatment vs. 20µM HCX. % P<0.05 Treatment vs.20µM HCX.
Figure 10. Oral microbial community in healthy periodontium (Control) and periodontitis (comparison 1). A Composition of oral microbial community at phylum level.
Figure 11. Relative abundance of phyla. Oral microbial community in treatment A (animals without treatment), treatment B (animals with HA+ 3mM HCX gel treatment) and treatment C (animals with HA+ 0.2% CHX gel treatment) (comparison 2).
Figure 12. Relative abundance (%) of the identified family in animals with treatment A (animals without treatment), treatment B (animals with HA with 3mM HCX gel treatment) and treatment C (animals with HA with 0.2% CHX (commercial gel) treatment). (A)Relative abundance (%) of Enterobacteriaceae. (B) Relative abundance (%) of Sphingomonadaceae. P values (<0.05) were obtained using Kruskal Wallis + Conover's test: *P<0.05 Treatment vs. treatment A. # P<0.05 Treatment vs. treatment B.

### DETAILED DESCRIPTION OF THE INVENTION

### Definitions

As used throughout, ranges are used as shorthand for describing each and every value that is within the range. Any value within the range can be selected as the terminus of the range. In addition, the compositions and the methods may comprise, consist essentially of, or consist of the elements described therein.

As used herein, "antimicrobial activity or properties" herein means activity as determined by any generally accepted *in vitro* or *in vivo* antibacterial assay or test.

An "oral surface" herein encompasses any soft or hard surface within the mouth including surfaces of the tongue, hard and soft palate, buccal mucosa, gums and dental surfaces. A "dental surface" herein is a surface of a natural tooth or a hard surface of artificial dentition including a crown, cap, filling, bridge, denture, dental implant and the like.

As used in the present invention, it is noted that 9-hydroxycalabaxanthone may be in the form of salts, solvates or stereoisomers, preferably pharmaceutically acceptable salts, solvates or stereoisomers.

As already indicated, the present invention also provides "salts" of 9-hydroxycalabaxanthone. By way of illustration, said salts can be acid addition salts, base addition salts or metal salts, and can be synthesized from 9-hydroxycalabaxanthone by means of conventional chemical processes known by the person skilled in the art. See, generally, G. S. Paulekuhn, et al., "Trends in Active Pharmaceutical Ingredient Salt Selection based on Analysis of the Orange Book Database", J. Med. Chem., 2007, 50: 6665-72, S. M. Berge, et al., "Pharmaceutical Salts", J Pharm Sci., 1977, 66:1-19, and Handbook of Pharmaceutical Salts, Properties, Selection, and Use, Stahl and Wermuth, Eds., Wiley-VCH and VHCA, Zurich, 2002. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of acid addition salts include inorganic acid addition salts such as, for example, hydrochloride, hydrobromide, hydroiodide, sulfate, pyrosulfate, bisulfate, sulfite, bisulfite, nitrate, phosphate, monohydrogen-phosphate, dihydrogenphosphate, meta-phosphate, pyrophosphate, etc., organic acid addition salts such as, for example, acetate, maleate, fumarate, citrate, oxalate, succinate, tartrate, malate, mandelate, methanesulfonate, p-toluenesulfonate, camphorsulfonate, propionates, decanoates, caprylates, acrylates, formates, isobutyrates, caproates, heptanoates, propiolates, malonates, succinates, suberates, sebacates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, lactates, y-hydroxybutyrates, glycolates, propanesulfonates, naphthalene-1-sulfonates, naphthalene-2-sulfonates, etc. Illustrative examples of base addition salts include inorganic base salts such as, for example, ammonium salts and organic base salts such as, for example, ethylenediamine, ethanolamine, N,N-dialkylenethanolamine, triethanolamine, glutamine, amino acid basic salts, etc. Illustrative examples of metal salts include, for example, sodium, potassium, calcium, magnesium, aluminum and lithium salts.

The term "solvate" according to this invention is to be understood as meaning any form of 9-hydroxycalabaxanthone which has another molecule (most likely a polar solvent) attached to it via non-covalent bonding. Examples of solvates include hydrates and alcoholates. Solvation methods are generally known in the state of the art.

As used herein, the term "stereoisomer" is a general term for all isomers of 9-hydroxycalabaxanthone that differ only in the orientation of their atoms in space. Therefore, stereoisomer compounds are molecules that are non-superimposable mirror images of each other and include enantiomers and diastereomers.

The terms "enantiomer" and "enantiomeric form" refer to one of the two stereoisomers of 9-hydroxycalabaxanthone that are mirror images of each other that are non-superimposable.

Enantiomer compounds are optically active, wherein one enantiomer rotates the plane of polarized light in one direction and the other one rotates the plane of polarized light in the opposite direction and form a racemic compound when present in equal quantities.

The term "racemic" or "racemate" refers to a mixture that has equal amounts of enantiomers and which mixture is optically inactive.

The terms "diastereomer" and "diastereomeric form" refer to stereoisomers of a compound with more than one chiral center that are not mirror images of one another.

The term "pharmaceutically acceptable" relates to molecular entities and compositions being physiologically tolerable and normally not causing an allergic reaction or similar adverse reaction, such as gastric discomfort, dizziness and the like, when they are administered to a human being. Preferably, as used in this description, the term "pharmaceutically acceptable" means approved by a governmental regulatory agency or listed in the US pharmacopoeia or another generally recognized pharmacopoeia for use in animals, and more particularly in humans.

### Description of embodiments

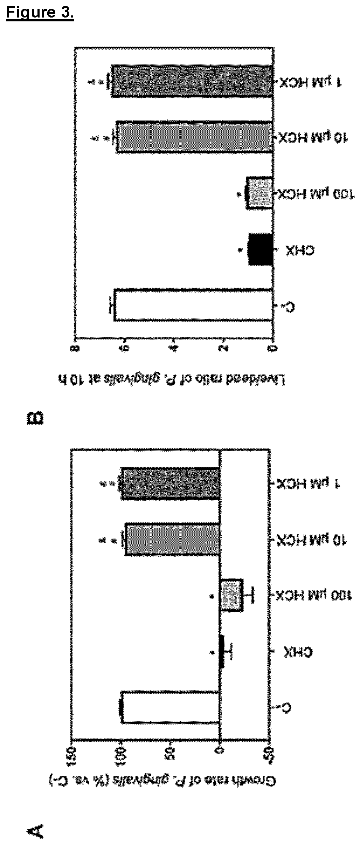

The present invention is confronted with the problem of providing an improved alternative antiseptic composition to chlorhexidine (CHX), that provides strong antimicrobial properties without cytotoxic effects. In this sense and as shown in example 1, in experiments with human gingival fibroblast cells, cytotoxicity evaluated by means of the activity of lactate dehydrogenase (LDH) released into the medium, was not observed when the cells where treated with 9-hydroxycalabaxanthone (HCX). These results also showed the antimicrobial properties of HCX with respect to P. gingivalis, wherein at a concentration of 100 µM bacterial growth rate was stopped, and the ratio of live/dead bacteria was strongly reduced (Figure 3).

That is, HCX has antimicrobial properties without cytotoxic effects on human gingival cells.

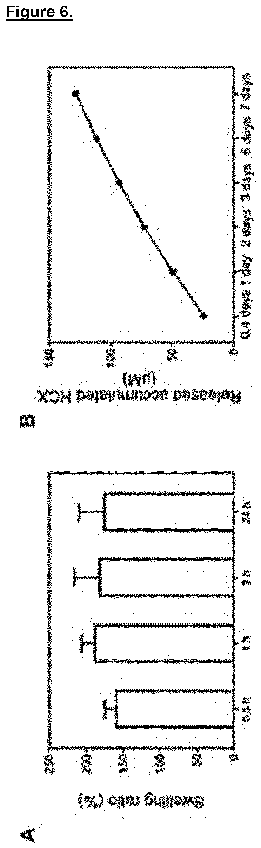

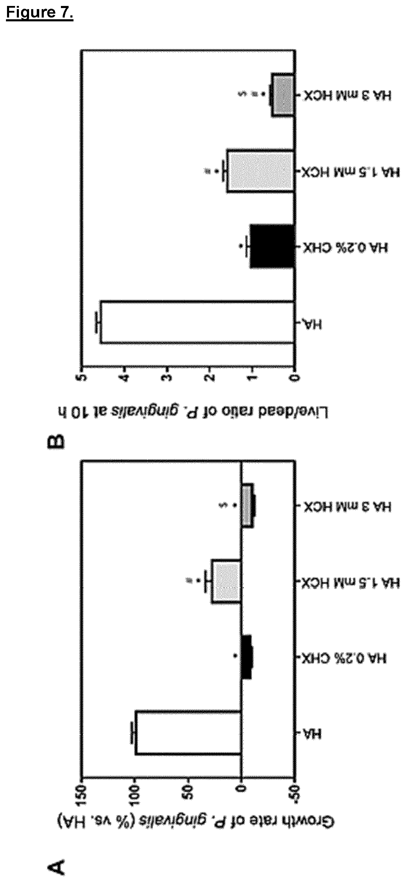

In addition, as shown in example 2, results of the formulation of the HA-HCX (hyaluronic acid- 9-hydroxycalabaxanthone) in the form of a hydrogel show that the modified HA hydrogel absorbs large amounts of water (Figure 6A) and that a controlled release of HCX into the aqueous medium occurs, maintaining a moderately sustained release of 20 µM after 10 h and up to 7 days (Figure 6B). The quantification of released HCX shows that the concentration released and accumulated in one week was 120 µM. As shown in example 3, the results, as illustrated in Figure 7, show that the formulation of the HA-HCX (hyaluronic acid- 9-hydroxycalabaxanthone) in the form of a hydrogel has good antimicrobial properties, with a higher inhibition for a concentration of 3 mM similar to the effect produced by a commercial gel containing 0.2% chlorhexidine and unmodified hyaluronic acid (Periokin Hyaluronic 1%, Laboratories Kin, Barcelona, Spain, HA 0.2% CHX).

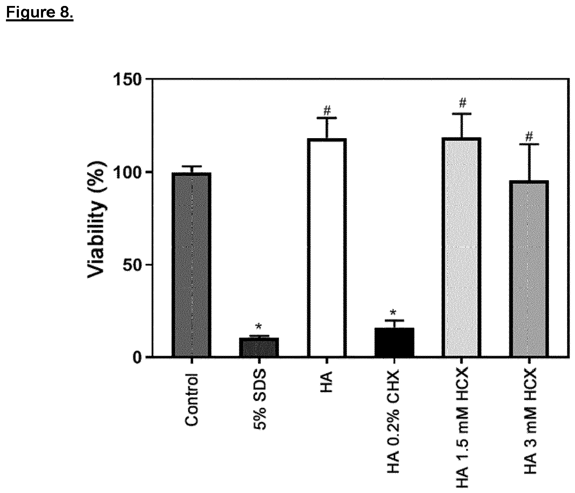

That is, again, HCX has good antimicrobial properties, moreover, hydrogels or gels containing HCX provide for a suitable antiseptic or antimicrobial composition for topical or oral administration in oral surfaces. Moreover, as shown in Figure 8 said hydrogels or gels containing HCX presents very good biocompatibility in a 3D gingival tissue, similar to modified HA without HCX and to an untreated gingival control tissue, for the two concentrations tested of 1.5 and 3 mM. This is in clear contrast to the commercial gel containing 0.2% chlorhexidine and unmodified hyaluronic acid (Periokin Hyaluronic 1%, Laboratories Kin, Barcelona, Spain, HA 0.2% CHX) which presents toxic effects on the tissue.

Furthermore, example 5 clearly shows that a concentration of 50 µM of HCX incubated for 10h on S. epidermidis is sufficient to stop bacterial growth rate and to kill the bacteria, which is similar to the effectivity of the most common antiseptic used for the skin, chlorhexidine.

Lastly and for the particular case of periodontitis the results of example 6 show that the HA gel containing HCX can help to re-stabilized the oral microbiome, by increasing the presence of bacteria related to a healthy oral microbiota, and by reducing the present of microbiota associated with periodontitis.

Therefore, the compositions of the present invention comprising 9-hydroxycalabaxanthone (HCX) inhibit the growth of various skin and oral bacteria that are implicated, for example, in forming plaque and causing oral diseases and skin diseases or skin infections in lesions such as burns or chronic wounds. Consequently, in a first aspect, the present invention, refers to a composition comprising 9-hydroxycalabaxanthone (HCX) or salts, solvates, stereoisomers or enantiomers thereof (from herein after referred to as the antiseptic composition of the present invention). Preferably, the antiseptic composition of the present invention is for use as a topical or oral antiseptic agent in a subject in need thereof at a concentration between 0.1 µM and 100 mM. Preferably, the composition comprises between 1 µM and 5 mM or 3mM, preferably between 1 µM, 10 µM, 25 µM, or 50µM and 0.5 mM, more preferably between 1 µM, 10 µM, 25 µM, or 50µM and 0.2 mM, more preferably between 1 µM, 10 µM, 25 µM, or 50µM and 0.15 mM, more preferably between 50 µM and about 0.1 mM, of 9-hydroxycalabaxanthone or salts, solvates, stereoisomers or enantiomers thereof (HCX) and is for use as a topical antiseptic agent. Also preferably, the composition comprises between 1 µM and 5mM or 3 mM, preferably between 1 µM, 10 µM, 25 µM, or 50µM and 0.5 mM, more preferably between 1 µM, 10 µM, 25 µM, or 50µM and 0.2 mM, more preferably between 1 µM, 10 µM, 25 µM, or 50µM and 0.15 mM, more preferably between 50 µM and about 0.1 mM, of 9-hydroxycalabaxanthone or salts, solvates, stereoisomers or enantiomers thereof (HCX) and is for use as an oral antiseptic agent. It is noted that when 9-hydroxycalabaxanthone or salts, solvates, stereoisomers or enantiomers thereof (HCX) is formulated as a hydrogel, concentrations of up 3 mM of this compound is included in the hydrogel as these types of formulations provide for a slow release profile. For other oral formulations, concentrations of between 50 µM and 0.1 mM are specially preferred.

In a preferred embodiment, the antiseptic composition of the present invention is for use in the prevention or treatment, via topical administration, of diseases of microbial origin in the skin such as those caused by Staphylococcus aureus, Staphylococcus epidermidis and/or Epidermolysis bullosa, preferably at a concentration between 1 µM and 5 mM or 3mM, preferably between 1 µM, 10 µM, 25 µM, or 50µM and 0.5 mM, more preferably between 1 µM, 10 µM, 25 µM, or 50µM and 0.2 mM, more preferably between 1 µM, 10 µM, 25 µM, or 50µM and 0.15 mM, more preferably between 50 µM and about 0.2mM or 0.1mM. In another preferred embodiment, the antiseptic composition of the present invention is for use in oral hygiene and/or for the prevention or treatment, via topical administration, of oral diseases of microbial origin such as those selected from the list consisting of periodontal diseases, plaque, caries, halitosis, gingivitis, mucositis, mycosis, and oral aphthous ulcers. Preferably, the disease is periodontitis and the related microbes are Porphyromonas gingivalis, Aggregatibacter actinomycetemcomitans, Tannerella forsythia, Treponema denticola or Fusobacterium nucleatum, preferably at a concentration between 1 µM and 5 mM or 3mM, preferably between 1 µM, 10 µM, 25 µM, or 50µM and 3 mM, preferably between 1 µM, 10 µM, 25 µM, or 50µM and 2 mM, preferably between 1 µM, 10 µM, 25 µM, or 50µM and 0.5 mM, more preferably between 1 µM, 10 µM, 25 µM, or 50µM and 0.2 mM, more preferably between 1 µM, 10 µM, 25 µM, or 50µM and 0.15 mM, more preferably between 50 µM and about 3 mM.

The antiseptic composition of the present invention may be thus topically applied to the skin, or to mucosae or to oral surfaces in the oral cavity. The antiseptic composition of the present invention promotes overall health, in particular oral health including preventing plaque formation, caries formation, calculus formation, halitosis, gingivitis, and periodontitis, for example. For example, as shown herein, in an embodiment of the present invention, where an oral care composition comprises an orally acceptable delivery-carrier, such as hyaluronic acid, and a safe and effective amount of HCX, the antibacterial activity is highly efficacious against both gram-positive and gram-negative bacteria.

As noted above, the antiseptic composition of the present invention comprises at least 9-hydroxycalabaxanthone (HCX) or salts, solvates, stereoisomers or enantiomers thereof, preferably at a concentration between 0.1 µM and 100 mM. Alternatively, the antiseptic composition of the present invention may comprise or consists of a sole active ingredient or component, namely 9-hydroxycalabaxanthone (HCX) or salts, solvates, stereoisomers or enantiomers thereof, at a concentration between 0.1 µM and 100 mM of concentration. The fact that the composition may consist of a sole active ingredient does not preclude the possibility of other components or ingredients being present such as excipients, adjuvants etc...

As it is understood in the art, the amount to be included of 9-hydroxycalabaxanthone (HCX) or salts, solvates, stereoisomers or enantiomers thereof in the the antiseptic composition of the present invention should be safe and effective e.g., microbial growth, at the target site of a host to be treated, without undue adverse side effects (such as toxicity, invitation, or allergic response), commensurate with a reasonable benefit/risk ratio when used in the manner of this invention. The specific "safe and effective amount" will vary with such factors as the particular condition being treated, the physical condition of the patient, the duration of treatment, the nature of concurrent therapy (if any), the specific dosage form to be used, the excipient employed, the solubility of the active ingredient therein, and the dosage regimen desired for the oral composition. At any rate, the composition preferably comprises between 1 µM and 5 mM or 3mM, preferably between 1 µM, 10 µM, 25 µM, or 50µM and 0.5 mM, more preferably between 1 µM, 10 µM, 25 µM, or 50µM and 0.2 mM, more preferably between 1 µM, 10 µM, 25 µM, or 50µM and 0.15 mM, more preferably between 50 µM and about 0.2mM or 0.1 mM, of 9-hydroxycalabaxanthone or salts, solvates, stereoisomers or enantiomers thereof (HCX) when used as topical antiseptic agent (directly on the skin or mucosae); or 1 µM and 5 mM or 3mM, preferably between 1 µM, 10 µM, 25 µM, or 50µM and 3 mM, preferably between 1 µM, 10 µM, 25 µM, or 50µM and 2 mM, preferably between 1 µM, 10 µM, 25 µM, or 50µM and 0.5 mM, more preferably between 1 µM, 10 µM, 25 µM, or 50µM and 0.2 mM, more preferably between 1 µM, 10 µM, 25 µM, or 50µM and 0.15 mM, more preferably between 50 µM and about 3 mM, of 9-hydroxycalabaxanthone or salts, solvates, stereoisomers or enantiomers thereof (HCX) when used as an oral antiseptic agent in the oral surface. Please note that depending on the formulation of the oral care product, the concentration shall vary.

In various embodiments, the antiseptic composition of the present invention is used to prepare oral or topical compositions such as, hydrogels, dentifrices, gels, creams, and mouth rinses. Preferably, the antiseptic composition of the present invention is used to prepare oral compositions in the form of a hydrogel, mouthwash, gel, toothpaste or other forms of oral or topical use.

Preferably, the antiseptic composition of the present invention is a cosmetic or pharmaceutical composition containing 9-hydroxycalabaxanthone (HCX) or salts, solvates, stereoisomers or enantiomers in the form of gel, aqueous solution, soap, or other presentations, which can be used efficiently in the antiseptic treatment of the buccal cavity, as well as in the disinfection of surfaces such as body surfaces, for example, in the treatment of medical conditions associated with infection (such as acute or chronic wounds, burns, and surgical wounds), or disinfection of the skin before a surgical procedure. As already indicated through-out the specification, the advantage of using HCX is that it has potent antimicrobial effects, in the absence of toxicity in living tissues, such as the skin (both healthy and compromised) such as mucosae, including oral mucosa and gums.

In various embodiments, the antiseptic composition of the present invention, at any of the concentrations above established, is a topical or oral care composition. However, additional antibacterial, antioxidant and/or ant inflammatory active ingredients may be included in the oral care compositions. If added, the antibacterial, active ingredients should not react with or detract from the efficacy and bioavailability of the 9-hydroxycalabaxanthone (HCX) or salts, solvates, stereoisomers or enantiomers thereof. Suitable antibacterial, anti-inflammatory, and/or antioxidant agents for use in addition to the 9-hydroxycalabaxanthone (HCX) or salts, solvates, stereoisomers or enantiomers thereof, iinclude any known in the art, botanical or synthetic, vitamins, proteinoid agents, peptides, minerals, salts and/or biologics.

Other natural extracts that are known antimicrobial agents are those listed in: the International Cosmetic Ingredient Dictionary and Handbook, Tenth Bd., 2004.

In various embodiments, the additional agents added to the oral composition of the present invention comprise from 0.0001% to 10%, preferably from 0.001% to 5%, more preferably from 0.01% to 3%, depending on the concentration of the active compounds and form of the oral composition.

In certain embodiments, the oral or topical compositions of the present invention optionally comprise one or more additional active ingredients. Such material may be operable for the prevention or treatment of a condition or disorder of hard or soft tissue of the oral cavity or of the mucosae or skin, for the prevention or treatment of a physiological, localized or systemic disorder or condition, or to provide a cosmetic benefit. Optional oral or topical care actives among those useful herein include antibacterial agents, antiplaque agents, anti-adhesion, antioxidant, anticaries agents, anti-inflammatory agents, desensitizing agents, whitening agents, tartar control agents, periodontal actives, nutrients, abrasives, breath freshening agents, malodour control agents, tooth desensitizers, salivary stimulants, and combinations thereof, such as those known to one of skill in the art.

Various optional oral care actives may be included in the oral composition of the present invention including those described above, such as antibacterial agents, antiplaque agents, anti-adhesion (that prevent adhesion of plaque to an enamel surface), antioxidant (such as Vitamin E or coenzyme Q10), anticaries agents, desensitizing agents (such as potassium citrate, potassium tartrate, potassium chloride, potassium sulfate and potassium nitrate), whitening agents (such as, urea peroxide, sodium percarbonate, sodium perborate and polyvinylpyrrolidone-H202); compatible enzymes; anti-inflammatory agents (such as, steroidal agents including flucinolone and hydrocortisone, and nonsteroidal agents (NSAIDs)), tartar control agents, periodontal actives, chlorophyll compounds, nutrients (such as vitamins, minerals, and amino acids, lipotropics, fish oil, coenzymes and the like) abrasives, breath freshening/malodour control agents (such as zinc salts such as zinc gluconate, zinc citrate, zinc chlorite, and α-ionone), and salivary stimulants (such as such as citric, lactic, malic, succinic, ascorbic, adipic, fumaric and tartaric acids); and any other suitable ingredients for oral care known to one of skill in the art.

In various embodiments, the oral compositions of the present invention comprise antitartar agents to prevent and/or minimize calculus formation. One or more of such agents can be present.

Suitable anticalculus agents include without limitation: phosphates and polyphosphates. Phosphate and polyphosphate salts are generally employed in the form of their wholly or partially neutralized water-soluble cationic species (e.g., potassium, sodium or ammonium salts, and any mixtures thereof). Thus, useful inorganic phosphate and polyphosphate salts illustratively include monovalent cations with monobasic, dibasic and tribasic phosphates; tripolyphosphate and tetrapolyphosphate; mono-, di-, tri- and tetrapyrophosphates; and cyclophosphates (also generally known in the art as "metaphosphates"). Useful monovalent cations of such phosphate salts include hydrogen, monovalent metals including alkali metals, and ammonium, for example.

Synthetic anionic polycarboxylates may also be used in the dentifrice compositions of the present invention as an efficacy enhancing agent for certain active ingredients, including antibacterial, antitartar or other active agents within the oral composition. Such anionic polycarboxylates are generally employed in the form of their free acids or preferably partially or more preferably fully neutralized water-soluble alkali metal (e.g. potassium and preferably sodium) or ammonium salts. As discussed above, preferred copolymers are of maleic anhydride or acid with another polymerizable ethylenically unsaturated monomer, preferably methylvinylether/maleic anhydride having an approximate molecular weight (M.W.) of about 30,000 to about 2,500,000 most preferably about 30,000 to about 2,000,000. Examples of these copolymers are available from ISP corporation under the tradename Gantrez, e.g. AN 139 (M.W. 1,100,000), AN 119 (M.W. 200,000); S-97 Pharmaceutical Grade (M.W. 1,500,000), AN 169 (M.W. 2,000,000), and AN 179 (M.W. 2,400,000); wherein the preferred copolymer is S-97 Pharmaceutical Grade (M.W. 1,500,000).

The anionic polycarboxylate, is employed in certain embodiments in amounts effective to achieve the desired enhancement of the efficacy of any antibacterial, antitartar or other active agent within the dentifrice composition, Generally, the anionic polycarboxylates is present within the dentifrice composition from 0.05% to, 5% by weight, preferably from 0.5% to 2.5% by weight.

Orally acceptable carriers for use in the invention include the usual components of hydrogels, toothpastes, tooth powders, prophylaxis pastes, mouth rinses, lozenges, gums and the like. Selection of specific carrier components is dependent on the desired product form, including dentifrices, rinses, gels, and confectionaries.

As recognized by one of skill in the art, the oral compositions of the present invention optionally include other materials, such as for example, viscosity modifiers, diluents, surface active agents, such as surfactants, emulsifiers, and foam modulators, pH modifying agents, abrasives, humectants, emollients, and moisturizers, mouth feel agents, sweetening agents, flavor agents, colorants, preservatives and combinations thereof. It is understood that while general attributes of each of the above categories of materials may differ, there may be some common attributes and any given material may serve multiple purposes within two or more of such categories of materials.

In the present invention, it is particularly preferred that for the oral, preferably dental, application thereof of the antiseptic composition of the present invention is a gel developed by means of the optimal combination of the biomolecule 9-hydroxycalabaxanthone (HCX) or salts, solvates, stereoisomers or enantiomers thereof and a hydrogel consisting of hyaluronic acid (HA) modified by means of chemical crosslinking or chemical gelling, allowing the controlled release of HCX, resulting in a gel with antibacterial and regenerative activity, that is biocompatible. Products currently found on the market have antibacterial activity but are toxic for tissue.

Native HA can be chemically functionalized to produce chemically reactive HA derivatives. Modifying HA consists of incorporating amino groups onto one batch of HA (HA-Amino) and creating aldehyde groups in another batch of HA (HA-Aldehyde) which react chemically when combined and are crosslinked to form the hydrogel which is relatively stable at physiological pH. One advantage of this system is that the precursor solutions of the gel can be applied as liquid to form the gel *in situ.* Furthermore, this configuration offers the possibility of formulating hydrogels with different mechanical properties by only varying the amounts or ratios of HA-Amino/HA-Aldehyde. With this approach, the results show that the HA hydrogel obtained is very stable, readily obtained in very mild conditions and at room temperature. The results show that despite the chemical modifications made on HA, its biocompatibility and regenerative properties are not lost, and it is not cytotoxic. On the other hand, and as already indicated, the antibacterial effect of 9-hydroxycalabaxanthone (HCX) in solution before incorporating same into HA has first been evaluated on *Porphyromonas gingivalis,* a common bacterium in periodontitis. The results indicate that, individually, 100 µM is sufficient to eliminate 100% of the bacteria. Experiments were also performed with human cells to verify the biocompatibility thereof, and the results indicate that at this concentration, i.e., 100 µM, there is a slight decrease in metabolic activity which is surprisingly reverted when combined with HA either due to an effect of the modified HA or else to the controlled release of HCX. Given that the precursor solutions of the modified HA hydrogel are prepared as aqueous solutions (water or PBS), a molecule can be incorporated into the gel provided that it can be dissolved or suspended in the aqueous solutions or provided that it is a water-miscible solution. With this modified HA hydrogel, a prolonged release system for HCX has been created. Therefore, based on a loading concentration of [HCX] = 3 mM, this system releases in a controlled manner a concentration of [HCX] = 20 µM after 10 h and sustained for at least 7 days, promoting a prolonged antimicrobial effect. Furthermore, in addition to the prolonged antibacterial activity, there are no cytotoxic effects on cells, and there is a regenerative effect due to the HA hydrogel.

It is noted that the present invention is not limited to gels of hyaluronic acid, although this is specially preferred, but any further hydrated gels are also suitable for the present invention. For example, further hydrogel gels useful in the present invention can be any type of hydrated gels that can be in turn classified according to their behavior towards water, into hydrophobic gels or lipogels and hydrophilic gels. Furthermore, according to the phases, they can be differentiated into single-phase gels or two-phase gels, or according to their viscosity into fluid, semi-solid or solid gels. According to the polymers used, gels useful in the present invention can be classified into:
- Natural:
   - Vegetable: i.e. gum arabic, gum karaya, gum tragacanth.
   - Seed extracts: i.e. guar gum, carob "carubin" gum, starch and derivatives, pectins.
   - Leaf and flower extracts: i.e. mallow mucilage, altea, calendula, plantain.
   - Marine origin: i.e. Agar-agar gum, Carrageenans (Irish moss), Alginates.
   - Animal origin: i.e. Casein, Gelatin
   - Exocellular hydrocolloids: Xanthan Gum
- Modified natural polymers:
   - Cellulose ester derivatives: i.e. Methylcellulose, Ethylcellulose, Ethylmethylcellulose, Ethylhydroxyethylcellulose, Hydroxyethylcellulose, Hydroxyethylmethylcellulose, Hydroxypropylcellulose, Hydroxypropylmethylcellulose, Sodium carboxymethylcellulose, Sodium sulfate cellulose, Carboxymethylhydroxyethylcellulose, Alkyl cellulose, Quaternized hydroxyethylcellulose.
   - Guar derivatives: i.e. hydroxy propyl guar (JAGUAR HP 8^{®}), carboxy methyl hydroxy propyl guar, hydroxy propyl trimonium guar chlorhydrate
- Vinyl polymers or copolymers: polyvinyl alcohols, polyvinyl pyrrolidone (PVP), PVP/PVA (polyvinyl acetate) and PVA/Ac. crotonic copolymers, polyvinyl methyl ether, methyl vinyl ether-malic anhydrous copolymer (MVE/MA), quaternary polyvinyls
- Carboxyvinyl polymers: carbomer (Carbopol^{®})
- Acrylic polymers - Acrylamides-: carboxypolyacrylic acid, methyl acrylate, ethyl acrylate, methyl methacrylate and methacrylic polymer, acrylic copolymer, acrylic interpolymer, polyacrylamide, dimethyl idantoinformaldehyde, imidazolinylurea
- Fatty acid esters of modified steroisomeric structure: 12-oxystearic triglycerides, aluminum hydroxystearate.
- Pyrogenic and precipitated silica anhydride - pyrogenic aluminum and titanium oxide: pyrogenic silica, precipitated silica, pyrogenic aluminum oxide, pyrogenic titanium oxide
- Alkaline earth silicate of colloidal lamellar structure or derivatives with quaternary traces for polymerization: bentonite, montmorillonite, atalpulguite, quaternized organophilic bentonite.

Therefore, in another preferred embodiment, the antiseptic composition of the present invention is in the form of a hydrogel, wherein the 9-hydroxycalabaxanthone or a salt thereof (HCX) is entrapped in the hydrogel.

Preferably, the antiseptic composition of the present invention is in the form of a hydrogel comprising water, a hyaluronic acid derivative and 9-hydroxycalabaxanthone or salts, solvates or stereoisomers, preferably pharmaceutically acceptable salts, solvates or stereoisomers thereof (HCX), wherein:
a. the hyaluronic acid derivative comprises hyaluronic acid, or salts, solvates or stereoisomers, preferably pharmaceutically acceptable salts, solvates or stereoisomers thereof, preferably of a molecular weight comprised between 50,000 and 3,500,000 Da;
b. the concentration of said hyaluronic acid derivative or salt thereof is preferably comprised between 0.001 % and 5 %; and
c. the 9-hydroxycalabaxanthone or salts, solvates or stereoisomers, preferably pharmaceutically acceptable salts, solvates or stereoisomers thereof (HCX) is entrapped in the hydrogel and has preferably a concentration comprised between 1 µM, 10 µM, 25 µM, or 50µM and 5 mM or 3mM, more preferably between 1 µM, 10 µM, 25 µM, or 50µM and 3.5 mM, more preferably between 1 µM, 10 µM, 25 µM, or 50µM and 3 mM, more preferably between 1 µM, 10 µM, 25 µM, or 50µM and 2 mM, more preferably between 1 µM, 10 µM, 25 µM, or 50µM and 1 mM, more preferably from between 1mM to 5 mM, more preferably from between 1mM to 3.5 mM, more preferably from between 1mM to 3 mM, more preferably about 3mM.

In another preferred embodiment, the antiseptic composition of the present invention, when present in the form of a hydrogel, the hyaluronic acid is at temperatures inducing gelling (physical crosslinking) and/or covalently crosslinked by chemically functionalized groups reactive to polymerization, preferably by nucleophilic addition reaction.

In another preferred embodiment, the antiseptic composition of the present invention, when present in the form of a hydrogel, the hyaluronic acid or derivative thereof is chemically functionalized to become reactive to polymerization or crosslinking, preferably by nucleophilic addition reaction.

In another preferred embodiment, the antiseptic composition of the present invention, when present in the form of a hydrogel, the hyaluronic acid or derivative thereof is crosslinked by the mix of two lots of hyaluronic acid where the first of them is functionalized preferably with adipic acid dihydrazide and the second of them is functionalized with aldehyde groups by the oxidation of vicinal hydroxyl groups, preferably with sodium periodate.

In various embodiments, and as already indicated, any of the oral compositions of the present invention, including the hydrogels, can be used in a method of promoting oral health in an oral cavity and for treating periodontal diseases, plaque, caries, halitosis, gingivitis, mucositis, mycosis, and oral aphthous ulcers, on an oral surface of a mammalian subject. Preferably, the disease is periodontitis and the related microbes are Porphyromonas gingivalis, Aggregatibacter actinomycetemcomitans, Tannerella forsythia, Treponema denticola or Fusobacterium nucleatum, preferably at a concentration of between 1 µM, 10 µM, 25 µM, or 50µM and 0.5 mM, more preferably between 1 µM, 10 µM, 25 µM, or 50µM and 0.2 mM, more preferably between 1 µM, 10 µM, 25 µM, or 50µM and 0.15 mM, more preferably between 50 µM and about 0.1 mM. The method comprises preparing an oral care composition comprising the antiseptic composition of the present invention. Then, the oral care composition is contacted with one or more oral surfaces of the oral cavity. In certain embodiments, when the oral composition is contacted with one or more oral surfaces in the oral cavity, the oral composition has the effect of reducing inflammation of oral tissue.

Thus, in various embodiments of the present invention, the oral care composition that is prepared is a hydrogel, gel, dentifrice, confectionary, or mouthwash, the oral composition is preferably applied regularly to an oral surface, preferably on a daily basis, at least one time daily for multiple days, but alternately every second or third day. Most preferably the oral composition is applied to the oral surfaces from 1 to 3 times daily, at a pH of 4.5 to 10, generally 5.5 to 8, preferably 6 to 8, for at least 2 weeks up to 8 weeks or more up to lifetime.

In other various embodiments, the antiseptic composition of the invention is for the application thereof on body surfaces such as the skin or mucosae, for example, in the treatment of medical conditions associated with infection such as acute or chronic wounds, burns, and surgical wounds, or disinfection of the skin before a surgical procedure, different HCX compositions are herein described for this purpose and their antimicrobial activity and biocompatibility tested in a healthy 3D skin tissue.

Finally, in other various embodiments, the antiseptic composition of the invention is for the application as a disinfectant formulation or solution for disinfecting a substrate surface. In this respect, the disinfectant formulation may be used for destroying microorganisms or a virus and/or inhibiting the growth of microorganisms or a virus on any substrate surface, such as a disinfecting solution for a soft contact lens. Commonly, the microorganisms are bacteria. The invention also relates to the disinfectant formulation or solution for use in a method of disinfecting and/or sterilizing any medical devices, contact lenses or any other device for use in the cosmetic or pharmaceutical industry, comprising treating the devices with the disinfectant solution of the invention.

As stated previously, the disinfectant formulation of the present invention may be suitable for disinfecting any substrate surface against a number of microorganisms including gram positive bacteria or gram-negative bacteria. The disinfectant formulation of the present invention may be applied to a substrate surface in any environment in which said surface is likely to come into contact with a microorganism or virus. Such environments include healthcare environments such as hospitals, clinics, and nursing homes, schools, sport facilities, hospitality, public transport, agriculture, manufacturing, residential homes, and cruise ships. Preferably, the disinfectant formulation is used in a healthcare environment such as a hospital. The substrate surface may be made of any material. In particular, substrate surfaces on which the disinfectant formulation of the present invention may be applied include wood, metal, laminates, plastics/polymer surfaces, ceramic etc.,

The following Examples further illustrate the present invention, but it is understood that the invention is not limited thereto. All amounts and proportions referred to herein and in the appended claims are by weight unless otherwise indicated.

### EXAMPLES

### Example 1. Evaluation of the antimicrobial activity of HCX on P. gingivalis and of the cytotoxicity thereof in gingival fibroblasts.

### MATERIAL AND METHODS

### 1.1. Different concentrations of HCX used

Three different concentrations of HCX (1 µM, 10 µM, and 100 µM) were used for studies with gingival fibroblasts and bacteria. Treated cells without the biomolecule served as negative control (C-) and chlorhexidine (CHX) (Abcam, Cambridge, United Kingdom) at 10 µM served as positive control.

### 1.2. Gingival fibroblast culture

Immortalized human gingival fibroblasts (iHGF) (Applied Biological Materials Inc, Richmond, BC, Canada) were cultured at 37°C in a 5% CO₂ atmosphere using a fibroblast medium consisting of low glucose Dulbecco's Modified Eagle Medium (DMEM) (Biowest, Nuaille, France) and Ham's F-12 medium (3/1) (Biowest), supplemented with 10% (v/v) embryonic stem cell fetal bovine serum (FBS) (Biowest) and 100 µg/ml penicillin, and 100 µg/ml streptomycin (Biowest). The culture medium was refreshed twice a week. Cells were seeded in 48-well plates at a density of 2 × 10⁴ cells/well. When the cells reached confluence, they were treated with different concentrations of HCX, and cytotoxicity was determined.

### 1.3. Treatment with different concentrations of HCX

48 hours after seeding the iHGF cells in 48-well plates, cell inflammation was induced by means of adding 1 µg/ml of P. *gingivalis* lipopolysaccharide (LPS) (InvivoGen, San Diego, CA, USA) and the cells were treated with different concentrations of HCX (1 µM, 10 µM, and 100 µM) in a fibroblast medium with 50 µg/ml of ascorbic acid (Sigma-Aldrich, St. Louis, MO, USA) for 48 hours. Images of the cells were taken with a bright field inverted microscope (Nikon Eclipse TS100) before treatment and 24 hours and 48 hours after treatment.

### 1.4. Cell cytotoxicity

To estimate the cytotoxicity of the different concentrations of HCX, the presence of lactate dehydrogenase (LDH) in the culture media 48 h after pro-inflammatory stimulation with LPS and treatment with HCX was used as the cell death index. Following the manufacturer's instructions (Cytotoxicity Detection kit, Roche Diagnostics, Mannheim, Germany), LDH activity was determined spectrophotometrically after a 30-minute incubation at room temperature (RT) of 50 µl of culture media and 50 µl of the reaction mixture, measuring nicotinamide adenine dinucleotide (NADH) oxidation at 490 nm in the presence of pyruvate. Results were presented in relation to LDH activity of the media of the cells seeded without treatment (negative control, 0% cell death) and in relation to cells treated with 1% Triton X-100 (positive control, 100% death) using the following equation: Cytotoxicity (%) = [(expected value - negative control) / (positive control - negative control)] x 100. The assays were performed in triplicate, with two replicates in each condition (n = 6).

### 1.5. Metabolic activity of the cells

Total metabolic activity was evaluated after treating the cells for 48 h with different concentrations of HCX. Presto Blue reagent (Life Technologies, Carlsbad, CA) Was used, incubating the reagent for 1 h following the manufacturer's protocol. Untreated cells were set as 100% cell viability. The assays were performed in triplicate, with two replicates in each condition (n = 6).

### 1.6. Bacterium P. gingivalis culture and proliferation assay

Bacterium P. *gingivalis* (ATCC 33277TM, Manassas, VA) was cultured in brain heart infusion (BHI) broth (Scharlab, Barcelona, Spain), supplemented with 0.5 g/I of L-cysteine hydrochloride (Thermo Fisher Scientific), 5.0 mg/l of hemin (Thermo Fisher Scientific), and 1.0 mg/l of vitamin K (Thermo Fisher Scientific) in anaerobic conditions (10% H₂, 10% CO₂, and 80% N₂) achieved with an anaerobiosis sachet and jar (Oxoid Anaerogen^{™}, Thermo Fisher Scientific) at 37°C for 24-72 h.

Bacteria P. *gingivalis* were cultured from existing frozen bacteria in BHI broth. After one night of incubation, 1 ml of bacterial suspension (≈ 3 × 10⁸ bacteria/ml) was treated with different concentrations of HCX (1 µM, 10 µM, and 100 µM) for 10 hours in anaerobic conditions (10% H₂, 10% CO₂, and 80% N₂) at 37°C. At regular intervals (0 h and 10 h), optical density (OD) was measured at 600 nm to determine bacterial proliferation (PowerWave Ht, Biotekinstruments, Winooski, VT). Bacterial growth rate (µ) was calculated for the exponential phase of growth following the equation In ODt - In OD₀ = µ- (t - t₀); the assay was performed in triplicate, with two replicates in each condition (n = 6). The proportion of living bacteria/dead bacteria was determined using the LIVE/DEAD BacLight bacterial viability kit (Invitrogen, Thermo Fisher Scientific, Waltham, MA, USA), following the manufacturer's instructions. The assays were performed in triplicate, with two replicates in each condition (n = 6).

### RESULTS

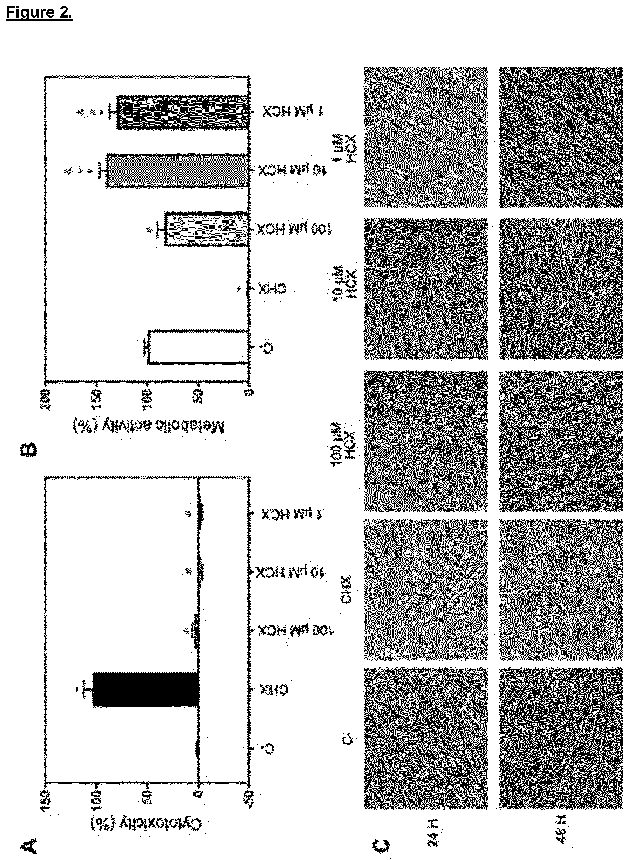

Individually, in experiments with human gingival fibroblast cells, cytotoxicity evaluated by means of the activity of lactate dehydrogenase (LDH) released into the medium is not observed (Figure 2).

Results of the evaluation of the antimicrobial properties of HCX with respect to P. *gingivalis* show that a concentration of 100 µM is sufficient to eliminate 100% of the bacteria (Figure 3).

### Example 2. Production of modified HA and incorporation of HCX

### MATERIAL AND METHODS

### 2.1 Synthesis of the HA-amino (HA-ADH) derivative

In a typical reaction, 500 mg (1.32 mmol; Mw = 1.2 × 10⁶ g/mol) of HA were dissolved in milliQ H₂O in a concentration of 3 mg/ml at room temperature. A 30-fold molar excess of adipic acid dihydrazide (ADH) (6887.9 mg; 39.54 mmol) was then added, and when it was completely dissolved, the pH of the reaction mixture was adjusted to 6.8 with solutions consisting of [NaOH] = 0.1 M and [HCI] = 0.1 M. A mixture formed by 1012.2 mg (5.28 mmol) of EDC/735.5 mg (5.28 mmol) of 1-hydroxybenzotriazole (HOBt) dissolved in 5 ml of DMSO/H₂O 1:1 was then added. The reaction was carried out overnight in darkness at room temperature and controlling the pH = 6.8 by means of adding [NaOH] = 0.1 M. After the reaction time, the pH was adjusted to 7.0 and the product was subjected to dialysis at 37°C for 7 days. NaCl was then added to the product until having a 5% solution (w/v), and the product was precipitated using three equivalent volumes of ethanol. The precipitate was then recovered, redissolved in milliQ H₂O at a concentration of 5 mg/ml, and lyophilized for one week. Finally, the product was stored at -70°C under N₂ atmosphere.

### 2.2 Synthesis of the HA-aldehyde (HA-CHO) derivative

In a typical reaction, 1000 mg (2.64 mmol; Mw = 1.2 × 10⁶ g/mol) of HA were dissolved in milliQ H₂O at a concentration of 3 mg/ml at room temperature. Next, 21 ml of a solution in milliQ H₂O of 563.9 mg (2.64 mmol) of sodium periodate (NaIO₄) were added dropwise. The reaction was carried out for 2 h at room temperature in darkness. Next, 671 µl (12 mmol) of ethylene glycol were added to inactivate any unreacted periodate. The solution was subjected to dialysis for one week and lyophilized for one week. The product was stored at -70°C under N₂ atmosphere.

### 2.3 Synthesis of HA hydrogels with HCX

Two solutions of [HCX] = 1.5 mM and 3 mM in milliQ H₂O were prepared. Then, HA-ADH and HA-CHO were dissolved separately in each of the aqueous solutions of [HCX] at a concentration of 7 mg/ml each. Hydrogels with HCX were formed by mixing the HA-ADH and HA-CHO solutions.

### RESULTS

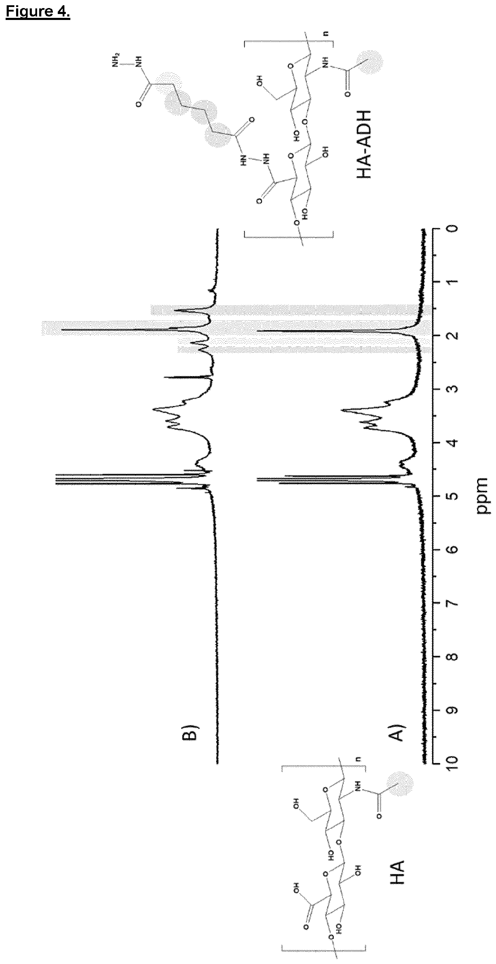

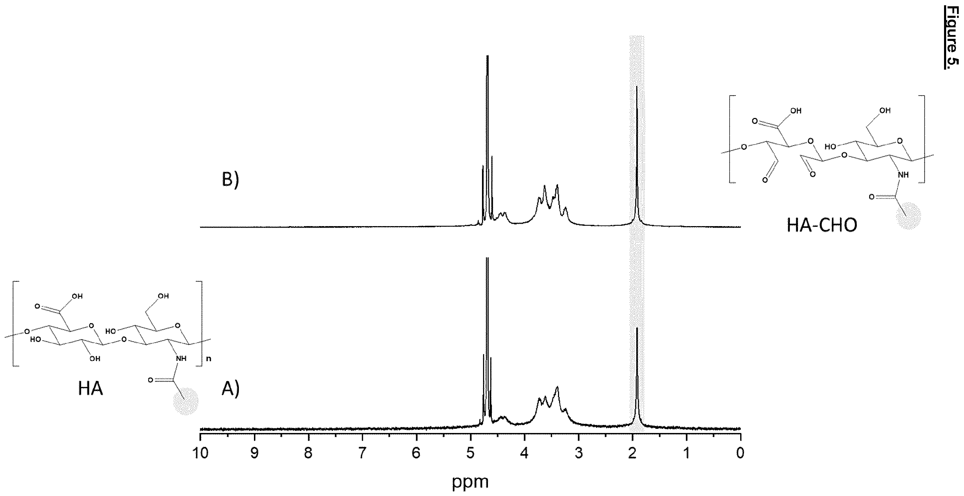

The bars of Figure 4 show the signals characteristic of the functional groups present in HA before and after chemical modification with ADH (Figure 3A and Figure 3B). HA is a polymer with a very high molecular weight (Mw = 1.2 × 10⁶ g/mol), hence the existence of the broad multiplet between about *δ* = 3.0-3.9 ppm which corresponds to the signal of the protons of the sugar rings. The signal a *δ* = 1.89 ppm highlighted by the green bar corresponds to the N-acetyl functional group of methyl and was used as the internal standard for calculating the degree of modification (DM) of the HA since this signal is rarely modified during the synthesis of HA derivatives. In this case, the DM was 45%, a good result taking into account the high molecular weight of the polymer of the present invention. Signals at *δ* = 1.53 ppm, *δ* = 2.14 ppm, and *δ* = 2,27 ppm highlighted by red, orange, and purple bars, respectively, correspond to the methylene groups of ADH and confirmed the successful chemical modification of HA by ADH. Furthermore, through NMR experiments for HA and HA-CHO (Figure 5), small changes were able to be confirmed in the region of the protons of the sugar rings, which allow confirming a new chemical structure in HA. Furthermore, the viscosity of the reaction mixture decreased visually during the reaction time, indicating the oxidation of HA and the slight loss of molecular weight typical in these processes.

Results of the formulation of the HA-HCX hydrogel show that the modified HA hydrogel absorbs large amounts of water (Figure 6A) and that a controlled release of HCX into the aqueous medium occurs, maintaining a moderately sustained release of 20 µM after 10 h and up to 7 days (Figure 6B). The quantification of released HCX shows that the concentration released and accumulated in one week is 120 µM.

### Example 3. Effect of the modified HA hydrogel with HCX on the growth and viability of P. gingivalis

### MATERIAL AND METHODS

### 3.1. Different concentrations of HA formulated with HCX.

Two different concentrations of HA formulated with HCX (1.5 mM and 3 mM) were used in the present study. Cells treated with HA without biomolecule served as negative control (HA) and cells treated with the commercial gel Periokin Hyaluronic 1% (Laboratorios Kin, Barcelona, Spain) containing 0.2% CHX and 1% HA (HA 0.2% CHX) served as positive control. Gels at a concentration of 5% (v/v) were used for bacterial studies.

### 3.2. Bacterium P. gingivalis culture and proliferation assay with different concentrations of modified HA with HCX.

The bacteria *P. gingivalis* were cultured and treated with different concentrations of HA formulated with HCX (1.5 mM and 3 mM), like in experiment 1 of section 1.6.

### RESULTS

The results of Figure 7 show that the gel modified with HCX has good antimicrobial properties, with a higher inhibition for a concentration of 3 mM and is similar to the effect produced by a commercial gel containing 0.2% chlorhexidine and unmodified hyaluronic acid (Periokin Hyaluronic 1%, Laboratories Kin, Barcelona, Spain, HA 0.2% CHX).

### Example 4. Effect of the modified HA hydrogel with HCX on the viability of a 3D gingival tissue

### MATERIAL AND METHODS

### 4.1. Cell culture

Immortalized human gingival keratinocytes (iHGK) (Applied Biological Materials Inc) were cultured in culture flasks for sensitive adherent cells (Sarstedt, Germany) at 37°C in a 5% CO₂ atmosphere using a keratinocyte medium consisting of calcium and magnesium-free DMEM (Gibco, Grand Island, NY, US)/Ham's F12 (3/1) (Biowest), supplemented with 0.01 mg/ml of insulin (Sigma-Aldrich), 0.4 ng/ml of hydrocortisone (Sigma-Aldrich), 6.7 ng/ml of selenium (Sigma-Aldrich), 0.01 µg/ml of human epithelial growth factor (Thermo Fisher Scientific, Waltham MA, USA), 1 M HEPES buffer (Biowest), 5.5 µg/ml of transferrin (Sigma-Aldrich), 10⁻¹⁰ M of cholera toxin (Sigma-Aldrich), 2 mM of L-glutamine (Sigma-Aldrich), 5% (v/v) of FBS (Biowest), and 100 µg/ml of penicillin, and 100 µg/ml of streptomycin (Biowest). The culture medium was refreshed twice a week.

iHGFs were cultured as described in Experiment 1, section 1.2. Both iHGK cultures and iHGF cultures with 70-80% confluence were used for constructing 3D tissue, as described in section 4.2 below.

### 4.2. 3D gingival tissue construction

3D gingival tissue was constructed according to the technique described by Dongari Bagtzoglou and Kashleva. In summary, a rat tail collagen type I solution (Thermo Fisher Scientific) (2.2 mg/ml) was mixed with iHGFs (1 ×10⁵ cells/well) and pipetted into a 24-well insert (Sarstedt) with pores measuring 0.4 mm. The collagen with fibroblasts was cultured at 37°C, 5% CO₂, for 7 days in a fibroblast medium. Next, iHGKs were seeded (2.5 × 10⁵ cells/well) on top of 3D tissues and cultured in a keratinocyte medium for 3 days at 37°C, 5% CO₂. Next, the 3D tissues were cultured in the air-liquid interphase at 37°C, 5% CO₂, for 15-17 days in an airlift (AL) culture medium consisting of low glucose DMEM (Biowest)/Ham's F12 (3/1) (Biowest), supplemented with 5 µg/ml of insulin (Sigma-Aldrich), 0.4 µg/ml of hydrocortisone (Sigma-Aldrich), 2 × 10⁻¹¹ M of 3,3',5-triiodo-L-thyronine (T3) (Sigma-Aldrich), 1.8 × 10⁻⁴ M of adenine (Sigma-Aldrich), 5 µg/ml of transferrin (Sigma-Aldrich), 10⁻¹⁰ M of cholera toxin (Sigma-Aldrich, St. Louis, MO, USA), 2 mM of L-glutamine (Sigma-Aldrich), 5% (v/v) of FBS (Biowest), and 100 µg/ml of penicillin, and 100 µg/ml of streptomycin (Biowest). The AL medium was changed every two days. After 25 days, the tissues were treated with modified HA gels with HCX.

### 4.3. Treatment of 3D gingival tissues with modified HA gels with HCX.

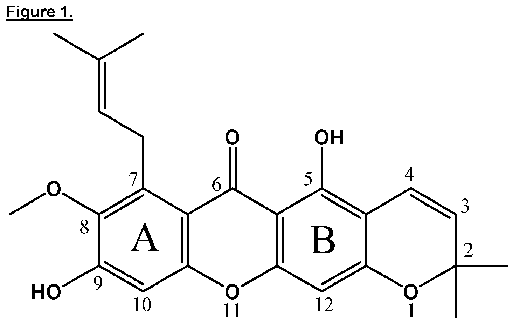

At the end of the 3D tissue incubation period, the tissues were surface treated with 50 µl of 1 µg/ml of P. *gingivalis* LPS (InvivoGen) for 24 h (Figure 1B) to cause inflammation therein. Next, 30 µl of HA gel, 30 µl of HA with 1.5 mM of HCX (45 nMol of HCX), 30 µl of HA gel with 3 mM of HCX (90 nMol of HCX), 30 µl of PBS (Biowest) (negative control), and 30 µl of the commercial gel Periokin (HA 0.2% CHX) (118.7 nMol of CHX) (positive control) were applied on the tissue for 48 h. The assays were performed in triplicate, with two replicates in each condition (n = 6).

### 4.4. MTT assay

At the end of the incubation period with different HA gels, the tissues were washed with PBS (Biowest) and incubated with 400 µl of 0.5 mg/ml of MTT (3-(4,5-dimethylthiazol-2-yl)-2,5-diphenyltetrazolium bromide (Thermo Fisher Scientific). After 3 hours of incubation at 37°C and 5% CO₂, the cultures were incubated with 2 ml of isopropanol (Sigma-Aldrich) for 24 h at RT. Optical density was measured with 200 µl of the extracts at 570 nm (reference filter: 690 nm). The negative control was obtained from the tissue culture media treated with PBS (100% viability). The positive control was obtained from the tissue culture media treated with 10% sodium dodecyl sulfate (SDS) diluted in PBS (1:1). Results are expressed as percentage of viability compared to the negative control. The assays were performed in triplicate, with two replicates in each condition (n = 6).

### RESULTS

The results of Figure 8 show that the modified gel with HCX presents very good biocompatibility in a 3D gingival tissue, similar to modified HA without HCX and to an untreated gingival control tissue, for the two concentrations of 1.5 and 3 mM. However, the commercial gel containing 0.2% chlorhexidine and unmodified hyaluronic acid (Periokin Hyaluronic 1%, Laboratories Kin, Barcelona, Spain, HA 0.2% CHX) presents toxic effects on the tissue, similar to the 5% SDS group used as positive control of 100% cell death.

### Example 5: Evaluation of the antimicrobial activity of HCX on Staphylococcus epidermidis

### MATERIAL AND METHODS

### 5.1. Culture and proliferation assay of S. epidermidis

S. epidermidis is a Gram-positive bacterium normally found on human skin and mucosa membranes. This bacterium can cause wound infections, boils, sinus infections, endocarditis, and other infections. S. epidermidis can survive for a long time on various surfaces - especially on polymeric plastic or metal - and form a biofilm that adheres well.

S. epidermidis 4814 (CECT, Valencia, Spain) were grown from frozen stocks in Luria-Bertani (LB) broth (Scharlab, Sentmenat), at 37 _{°}C under aerobic conditions in an orbital shaker (180 rpm). After an overnight incubation, 1 mL of bacterial suspensions were incubated with different concentrations of HCX (200, 100, 50, 20, 10 and 1 µM) for 24 hours at 37 _{°}C under aerobic conditions in an orbital shaker (180 rpm). Bacterial suspension without treatment served as negative control, and CHX at 10 µM served as positive control for bacterial growth inhibition. The optical density (OD) was measured at 600 nm at 0 h, 10h and 24 h to determine bacterial proliferation (PowerWave Ht, Biotek instruments, Winooski, VT, USA). Bacterial growth rate (µ) was calculated during the exponential growth phase following the equation In ODt - In OD0 = µ × (t - t0). Live/Dead ratio of bacteria was determined using the LIVE/DEAD BacLight bacterial viability kit (Invitrogen, Thermo Fisher Scientific, Waltham, MA, USA), following manufacturer's instructions. The assays were carried out in triplicate, with two replicates at each condition (n=6).

### RESULTS

Some of the most common infectious diseases are caused by bacteria that naturally colonize human skin asymptomatically. Staphylococcus epidermidis is carried asymptomatically on the skin and mucous membranes of virtually all humans but is a major cause of nosocomial infection associated with invasive procedures. In this example, we show that a concentration of 50 µM of HCX incubated for 10h on S. epidermidis is sufficient to eliminate 100% of bacteria and a concentration of 200 µM of HCX incubated for 24h on S. epidermidis eliminates 80% of bacteria, which is similar to the effectivity of the most common antiseptic used for the skin, chlorhexidine.

### Example 6: Effect of hyaluronic acid gel containing 3mM HCX on the bacteria present in the gingival crevicular fluid after inducing periodontitis in rats with lipopolysaccharide (LPS) from Porphyromonas gingivalis.

### MATERIAL AND METHODS

### 6.1. In vivo experimental protocol

In this study, Wistar rats aged 12 weeks and weighed 292-360 g were used. Experimental periodontitis was induced in all rats. A mixture of ketamine (anesketin) /xylazine (xylasol) 80/10 mg/kg was administered intraperitoneally to induce anaesthesia. Anaesthesia was maintained with 2-3% isoflurane in 100% oxygen using a small nose cone. The animal was placed on its back and the maxilla and mandible was stabilized in an open position with aluminium mouth gags. The ligature model was created by placing a sterile braided silk ligature (5/0) (Laboratorio Aragó, Barcelona, Spain) around the first maxillary molar bilaterally within the gingival sulcus using forceps and securing with the surgeon's knots on the lingual surface. Also, periodontitis was induced by injection of 40 µL of LPS (1mg/mL) (InvivoGen, San Diego, CA, USA) derived from P.Gingivalis in sterile saline, was injected 3 times per week with a microsyringe to the subgingival tissue at the mesio-palatal side of the first and second maxillary molars. The induction of periodontitis was maintained for 14 days to promote the accumulation of bacteria biofilm and consequent inflammation. Once the intervention was over, its effect was reversed with the antagonist atipamezole (antisedan 0.5 mg/kg) administered subcutaneously.

After 14 days of inducing periodontitis in the rats, animals were randomly divided into three treatment groups. Control was the group with no treatment (treatment A), HA with 3mM of HCX gel applied group (treatment B) and HA with 0.2% of CHX commercial gel applied group (treatment C, PERIOKIN HYALURONIC 1 % Gel). To be able to treat the animals with periodontitis with the different gels, the rats were under general anaesthesia. A mixture of ketamine (anesketin) /xylazine (xylasol) 80/10 mg/kg was administered intraperitoneally to induce anaesthesia. Anaesthesia was maintained with 2-3% isoflurane in 100% oxygen using a small nose cone. The animal was placed on its back and the maxilla and mandible must be stabilized in an open position with aluminium mouth gags. First, the ligatures were removed, and the surgical area was cleaned with saline. Next, an intracrevicular incision was made around a maxillary first molar with a microsurgical blade. The incision was extended to the mucosa of the anterior maxilla from the mesial surface of the molars to the posterior border of the incisive papilla. A full-thickness mucoperiosteal flap was raised. After hemostasis was achieved, 30µl of the gel was applied to the wound. The flaps were repositioned, and the wound was sutured with a sterile braided silk ligature (6/0) (Laboratorio Aragó). Once the intervention was over, its effect was reversed with the antagonist atipamezole (antisedan 0.5 mg/kg) administered subcutaneously. The gel treatments were carried out topically for the next 14 days, 3 times a week, 30 µl of the gels were homogeneously distributed with sterile swabs in the lingual and vestibular region around the molars, under isoflurane anaesthesia with 2-3% induction and 1% maintenance. Gingival Crevicular fluid (GCF) was collected before inducing periodontitis (basal control), before treatment at the onset of periodontitis, and after 14 days of treatment. GCF was collected using adsorbent paper point n°30 (Proclinics. S.A.U, Barcelona, Spain) around mesio-palatal of the fist molar. After collection, the paper point was immediately transferred into a plastic vial and stored at -80°C until the assays. After 28 days of the study, the animals were sacrificed with CO2 (>70%) in carbon dioxide chamber. Following euthanasia, samples of GCF were taken with adsorbent paper point (Proclinics. S.A.U) to perform cytokine analyses and a metagenomic characterization and comparative microbiota analysis of the different groups, then the jaws were extracted and fixed in formalin for radiological and histological analysis.

### 6.2. GCF DNA extraction, library preparation and sequencing.

The 16S rRNA gene analysis was performed at Microomics (Barcelona, Spain; http://www. microomics.eu/). DNA was extracted from adsorbent paper points of GCF at different times, before inducing periodontitis (basal control) and after inducting periodontitis for 14 days (comparison 1). DNA was also collected after 14 days of the application of different treatments (comparison 2) using MagMAX CORE Nucleic Acid Purification Kit 500 RXN (Thermo Fisher, CA, Austin), following the manufacturer's instructions. As a control for downstream procedures, Mock community DNA was included (Zymobiomics Microbial Community DNA, ZymoResearch, Irvine). Samples were amplified using 16S rRNA V3-V4 regions specific primers (V3-V4-Forward 50 - TCGTCGGCAGCGTCAGATGTGTATAAGAGACAGCCTACGGGNGGC WGCAG-30, V3-V4-Reverse 50 GTCTCGTGGGCTCGGAGATGTGTAT AAGAGACAGGACTACHVGGGTATCTAATCC-30).

The PCR was performed in 10 µL final volume with 0.2 µM primer concentration. The PCR cycle included: 3 minutes at 95°C (initial denaturation) followed by 25 cycles: 30 seconds at 95°C 30 s at 55°C, and 30 s at 72°C, and a final elongation step of 5 minutes at 72°C. PCR products were purified using AMPure XP beads (Beckman Coulter, Nyon, Switzerland) with a 0.9x ratio according to manufacturer's instructions. The above-described primers contain overhangs allowing the addition of full-length Nextera barcoded adapters for multiplex sequencing in a second PCR step, resulting in sequencing ready libraries with approximately 450 bp insert sizes. In brief, 5 µL of the first PCR purified product were used as template for a second PCR with Nextera XT v2 adaptor primers in a final volume of 30 µL using the same PCR mix and thermal profile as for the first PCR but with only 8 cycles. Twenty-five microliters of the second PCR product were purified with SequalPrep normalization kit (Invitrogen, ThermoFisher Scientific, Waltham, Massachusetts), according to the manufacturer's protocol. Libraries were eluted in 20 µL final volume and pooled for sequencing. The final pool was quantified by qPCR using Kapa library quantification kit for Illumina Platforms (Kapa Biosystems, SigmaAldrich, Saint Louis, Missouri) on an ABI 7900HT real-time cycler (Applied Biosystems, ThermoFisher Scientific, Waltham, Massachusetts). Sequencing was performed using Illumina MiSeq (2 × 300 bp) and v3 chemistry with a loading concentration of 10 PM In all cases, 15% of PhlX control libraries for run quality monitoring were used. Negative controls of the sample collection buffer, DNA extraction, and PCR amplification steps were routinely performed in parallel, using the same conditions and reagents, PCR products after both PCR steps were visualized by electrophoresis gel (1.5% agarose) with SYBR Safe (ThermoFisher Scientific, Waltham, Massachusetts).

### 6.3. Bioinformatics processing and statistical analysis.

Raw demultiplexed forward and reverse reads were processed using the following methods and pipelines as implemented in QIIME2 version 2019.4 with default parameters unless stated. DADA2 was used for quality filtering, denoizing, pair- end merging and amplicon sequence variant calling (ASV) using qiime dada2 denoise-paired method. Q20 was used as quality threshold to define read sizes for trimming before merging (parameters: - p-trunc-len-f and -p-trunclen-r). Reads were truncated at the position when the 75th percentile Phred score felt below Q20: 287 bp for forward reads, and 224 bp for reverse reads. After quality filtering steps, average sample size was 27240 (comparison 1) and 14200 (comparison 2) reads and 999 (comparison 1) and 2591 (comparison 2) phylotypes were detected. ASVs were aligned using the qiime alignment mafft method. The alignment was used to create a tree and to calculate phylogenetic relations between ASVs using the qiime phylogeny fasttree method. ASV tables were subsampled without replacement in order to even sample sizes for diversity analysis using qiime diversity core-metrics-phylogenetic pipeline. ASV tables were used to calculate alpha diversity metrics: community richness (ASVs), Evenness (or Pielou's Evenness; a measure of community evenness) and Shannon's diversity index (quantitative measure of community richness). Alpha diversity comparisons were performed using Kruskal Wallis non-parametric test. ASV tables and phylogenetic data were used to calculate beta diversity metrics: Bray-Curtis (non-phylogenetic quantitative measure) and Jaccard (non-phylogenetic qualitative measure) dissimilarity matrices, as well as Unweighted Unifrac (phylogenetic qualitative measure) and Weighted Unifrac (phylogenetic quantitative measure) distance matrices. Principal coordinated analysis (PCoA) was the method used for exploring and visualizing similarity of data using the matrices described above. The significance of groups present in community structure was tested using analysis of similarities (ANOSIM) and Permanova tests. Permdisp test was used to identify location vs dispersion effects. Distance-based redundancy analysis (dbRDA) was used to explore which variables constrained PCoA ordinations. Model selection was done stepwise using forward direction and a permutation test using vegan package version 2.5-5. Taxonomic assignment of ASVs was performed using Bayesian Classifier trained with Silva database (i.e., 99% OTUs database). Differential abundance of taxa was tested using two methods: ANCOM and Kruskal Wallis non-parametric test on relative abundance of taxa (total sum of scale). After Kruskal Wallis test, Conover's test was added for pairwise comparison. Benjamini-Hochberg correction was used to control false discovery rate (FDR). The relative abundance of each taxon was calculated as the number of 16S rRNA sequences of a given taxon divided by the total number of 16S rRNA sequence in a participant's sample. Significant threshold was set at 0.05. BiodiversityR version 2.11-1, PMCMR version 4.3, RVAideMemoire version 0.9-7 and vegan version 2.5-5 packages were used for the different stadistical analysis carried out.

### RESULTS

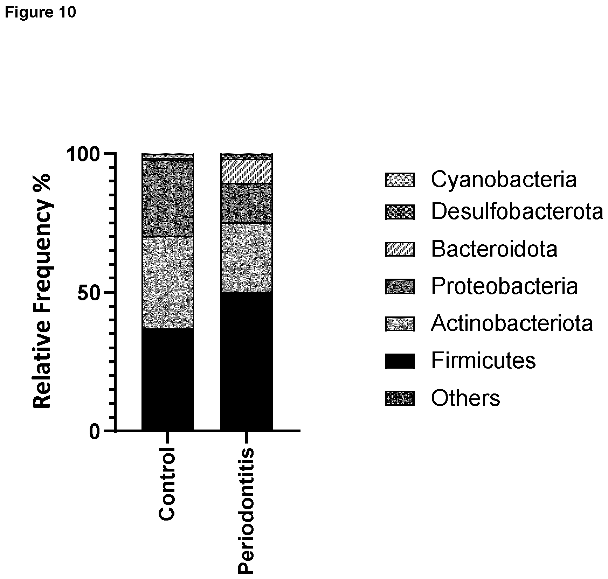

Figure 10 shows the most abundant Phyla in healthy periodontium and in periodontitis disease in rats. The most abundance phyla in both groups of animals were Firmicutes, Actinobacteria, Proteobacteria and Bacteroidetes. Rats with periodontal disease presented an increase in the percentage of relative frequency of Bacteroidetes and Desulfobacterota accompanied by reduction in actinobacteria and proteobacteria relative abundance. Thus, the results confirmed the complexity of the oral microbiome, and the alterations occurred at the phylum level in the oral microbiome of rats with periodontal disease. When this microbiome suffers certain imbalances, a dysbiosis state generates a favorable environment for pathogenic microorganisms, increasing the virulence of microorganisms present.

Periodontitis-associated dysbiosis is characterized by low abundance or absence of specific bacteria normally present in healthy subgingival microbiota, and also by the appearance of pathogenic bacteria.

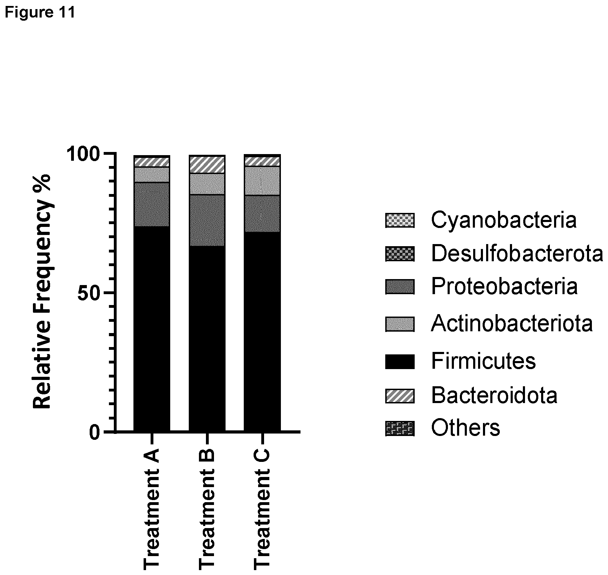

The oral microbiome also displayed differences in the most abundant phyla in periodontitis rats after applying the different treatments (A, B or C) (Figure 11). The most abundance phyla in all groups of animals were Firmicutes, Actinobacteria, Proteobacteria and Bacteroidetes.

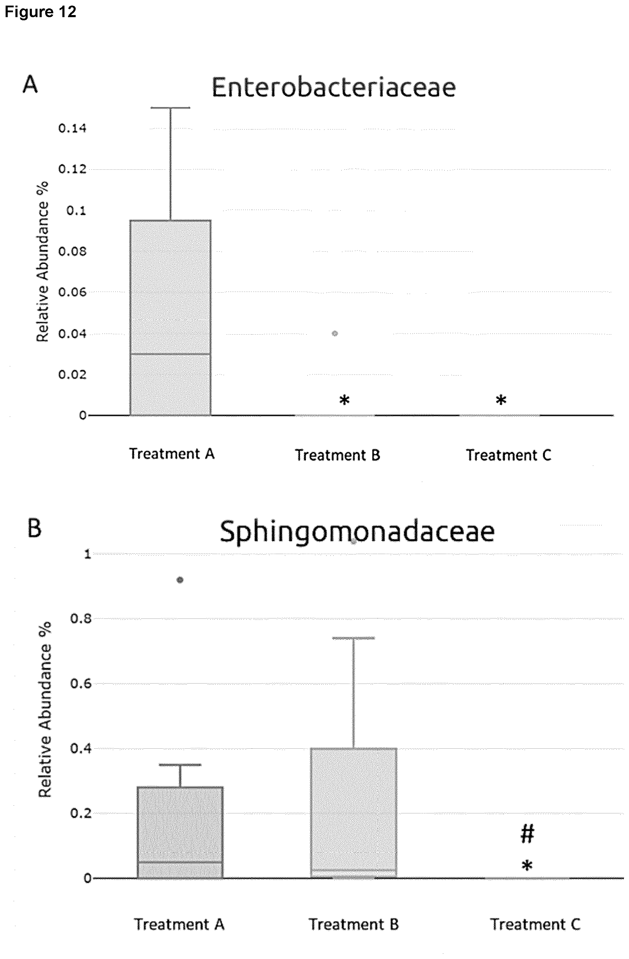

When comparing the different gel treatments, results demonstrate that Enterobacteriaceae (Figure 12A) was significantly increased in samples from Treatment A (Control) compared to treatment B (animals with HA+ 3mM HCX gel treatment) and C (animals with HA+ 0.2% CHX gel treatment). Microbiological studies in patients with periodontitis in different parts from the world have shown high prevalences of infection by Enterobacteriaceae, which could complicate the clinical picture of periodontal patients. On the other hand, Sphingomonadaceae, which is a specific bacterium present in the healthy subgingival microbiota (Figure 12B), was significant decreased in samples from Treatment C compared to treatment A and B.

Our results show the HA gel containing HCX can help to re-stabilize the oral microbiome, by increasing the presence of bacteria related to a healthy oral microbiota, and by reducing the present of microbiota associated with periodontitis.

## Claims

1. A composition comprising 9-hydroxycalabaxanthone (HCX) or a salt thereof between 0.1 µM and 100 mM of concentration for use as a topical or oral antiseptic agent.

2. The composition according to claim 1, wherein it comprises between 50 µM and 0.2 mM of 9-hydroxycalabaxanthone or a salt thereof (HCX) when used as a topical antiseptic agent.

3. The composition according to claim 1, wherein it comprises between 50 µM and 3 mM of 9-hydroxycalabaxanthone or a salt thereof (HCX) when used as an oral antiseptic agent.

4. The antiseptic composition for use according to claim 1 or 2, wherein the composition is for use in the prevention or treatment, via topical administration, of diseases of microbial origin in the skin such as those caused by *Staphylococcus aureus, Staphylococcus epidermidis* or *Epidermolysis bullosa.*

5. The antiseptic composition for use according to claim 1 or 3, wherein the composition is for use in oral hygiene and/or for the prevention or treatment, via topical administration, of oral diseases of microbial origin such as those selected from the list consisting of periodontal diseases, plaque, caries, halitosis, gingivitis, mucositis, mycosis, and oral aphthous ulcers.

6. The antiseptic composition for use according to claim 5, wherein the disease is periodontitis and the related microbes are *Porphyromonas gingivalis, Aggregatibacter actinomycetemcomitans, Tannerella forsythia, Treponema denticola or Fusobacterium nucleatum.*

7. The composition according to any of claims 1 to 6, wherein the composition is in the form of a cream, mouthwash, gel, hydrogel, toothpaste or other forms of oral or topical use.

8. The composition according to any of claims 1 to 6, wherein the composition is in the form of a pharmaceutical composition suitable for oral or topical use.

9. The composition for use according to any of claims 1 to 8, wherein the composition is in the form of a hydrogel, wherein the 9-hydroxycalabaxanthone or a salt thereof (HCX) is entrapped in the hydrogel.

10. The composition for use according to claim 9, wherein the composition is in the form of a hydrogel comprising water, a hyaluronic acid derivative and 9-hydroxycalabaxanthone or a salt thereof (HCX), wherein:
a. the hyaluronic acid derivative comprises hyaluronic acid, or a salt thereof, of molecular weight comprised between 50,000 and 3,500,000 Da;
b. the concentration of said hyaluronic acid derivative or salt thereof is comprised between 0.001 % and 5 %; and
c. the 9-hydroxycalabaxanthone or a salt thereof (HCX) is entrapped in the hydrogel and has a concentration comprised between 1 mM and 3 mM.

11. The composition for use according to claim 10, wherein the hyaluronic acid is at temperatures inducing gelling (physical crosslinking) and/or covalently crosslinked by chemically functionalized groups reactive to polymerization, preferably by nucleophilic addition reaction.

12. The composition for use according to any of claims 10 or 11, wherein the hyaluronic acid or derivative thereof is chemically functionalized to become reactive to polymerization or crosslinking, preferably by nucleophilic addition reaction.

13. The composition for use according to claim 12, wherein the hyaluronic acid or derivative thereof is crosslinked by the mix of two lots of hyaluronic acid where the first of them is functionalized preferably with adipic acid dihydrazide and the second of them is functionalized with aldehyde groups by the oxidation of vicinal hydroxyl groups, preferably with sodium periodate.

14. A composition in the form of a hydrogel, wherein a 9-hydroxycalabaxanthone or a salt thereof (HCX) is entrapped in the hydrogel.

15. A composition in the form of a hydrogel comprising water, a hyaluronic acid derivative and 9-hydroxycalabaxanthone or a salt thereof (HCX), wherein:
a. the hyaluronic acid derivative comprises hyaluronic acid, or a salt thereof, of molecular weight comprised between 50,000 and 3,500,000 Da;
b. the concentration of said hyaluronic acid derivative or salt thereof is comprised between 0.001 % and 5 %; and
c. the 9-hydroxycalabaxanthone or a salt thereof (HCX) is entrapped in the hydrogel and has a concentration comprised between 1 mM and 3 mM.
